Europäisches Patentamt

European Patent Office

Office européen des brevets

(11) Veröffentlichungsnummer: **0 498 316 B1**

(12) **EUROPÄISCHE PATENTSCHRIFT**

(45) Veröffentlichungstag der Patentschrift: **06.09.95**

(21) Anmeldenummer: **92101642.4**

(22) Anmeldetag: **31.01.92**

(51) Int. Cl.[6]: **C12P 17/12**, //(C12P17/12, C12R1:38),(C12P17/12, C12R1:07),(C12P17/12, C12R1:05)

(54) Mikrobielles Verfahren zur Herstellung von 6-Hydroxypicolinsäure.

(30) Priorität: **04.02.91 CH 330/91**

(43) Veröffentlichungstag der Anmeldung:
**12.08.92 Patentblatt 92/33**

(45) Bekanntmachung des Hinweises auf die Patenterteilung:
**06.09.95 Patentblatt 95/36**

(84) Benannte Vertragsstaaten:
**AT BE CH DE DK FR GB IT LI LU NL**

(56) Entgegenhaltungen:
**US-A- 4 859 592**

**CANADIAN JOURNAL OF MICROBIOLOGY, Band 20, Nr. 5, Mai 1974, Ottawa, ON (CD); R.L. TATE et al., Seiten 695-702**

**INDIAN JOURNAL OF BIOCHEMISTRY & BIO-PHYSICS, Band 10, Nr. 1, März 1973, New Delhi (IN); O.P. SHUKLA et al., Seiten 176-178**

(73) Patentinhaber: **LONZA AG**
**(Geschäftsleitung: 4002 Basel)**
**CH-3945 Gampel/Wallis (CH)**

(72) Erfinder: **Kiener, Andreas, Dr.**
**Meisenweg 5**
**Visp (Kanton Wallis) (CH)**
Erfinder: **Glöckler, Rainer**
**Biffigastrasse**
**Visperterminen (Kanton Wallis) (CH)**
Erfinder: **Heinzmann, Klaus**
**Wierastrasse**
**Visperterminen (Kanton Wallis) (CH)**

(74) Vertreter: **Weinhold, Peter, Dr. et al**
**Patentanwälte**
**Dr. Weinhold, Dannenberg,**
**Dr. Gudel, Schubert**
**Siegfriedstrasse 8**
**D-80803 München (DE)**

**Beschreibung**

Die Erfindung betrifft ein neues mikrobiologisches Verfahren zur Herstellung von 6-Hydroxypicolinsäure, ausgehend von Picolinsäure und/oder deren Salzen.

6-Hydroxypicolinsäure wird beispielsweise zur Herstellung von 2-Oxypyrimidin verwendet (Berichte der Deutschen Chemischen Gesellschaft, 1912, 45, S.2456-2467), welches wiederum ein wichtiges Zwischenprodukt zur Herstellung von Pharmazeutika ist.

Mehrere Methoden zur Herstellung von 6-Hydroxypicolinsäure mittels organischer Synthesen sind bekannt. Aus Picolinsäure kann bspw. die 6-Hydroxypicolinsäure durch Reaktion mit Kaliumhydroxid erhalten werden (Tetrahedron Letters, Vol.29, S.4389-4392, 1988).

Ein Nachteil dieses Verfahrens ist, dass die 6-Hydroxypicolinsäure nur in mässiger Ausbeute (51%) erhalten wird.

Bekannt ist auch, dass Mikroorganismen der Gattung Bacillus Picolinsäure zur 6-Hydroxypicolinsäure hydroxylieren (O. Shukla und S. M. Kaul, Indian J. of Biochemistry and Biophysics, Vol. 10, S. 176-178, 1973; O. Shukla et al., Indian J. of Biochemistry and Biophysics, Vol. 14, S. 292-295, 1977).

Ein grosser Nachteil dieses Verfahrens ist, dass die weitere Metabolisierung der 6-Hydroxypicolinsäure nur mit dem Inhibitor Natriumarsenit unterbunden werden kann, wobei damit auch das Wachstum der Mikroorganismen gehemmt wird.

Ein weiterer Nachteil besteht darin, dass nicht ausschliesslich 6-Hydroxypicolinsäure gebildet wird, sondern ein Gemisch von 3,6-Dihydroxypicolinsäure und 6-Hydroxypicolinsaure.

R. L. Tate und J. C. Ensign, Can. J. Microbiol., Vol. 20, S. 695-702, 1974, beschreiben die Hydroxylierung von Picolinsäure mit Mikroorganismen der Gattung Arthrobacter.

Nachteile dieses Verfahrens sind, dass diese Mikroorganismen Picolinsäure nicht ausschliesslich als Kohlenstoff-, Stickstoff- und Energiequelle nutzen können, sondern bei der Hydroxylierung Hefe-Extrakt anwesend sein muss, der zu unerwünschten Verunreinigungen des Produktes führen kann.

Ein weiterer Nachteil liegt darin, dass die 6-Hydroxypicolinsäure nur bei geringem Sauerstoffgehalt gebildet wird, wobei sich die Mikroorganismen nicht in der Wachstumsphase befinden und dadurch wenig Produkt gebildet wird.

Im folgenden werden unter Picolinsäure auch deren Salze, wie beispielsweise deren wasserlösliche Alkalisalze, Ammoniumsalze oder auch beispielsweise ein Gemisch aus Picolinsäure und deren wasserlösliche Alkalisalze, verstanden.

Aufgabe der vorliegenden Erfindung war es, die genannten Nachteile zu beseitigen und ein wirtschaftliches mikrobiologisches Verfahren zur Herstellung von 6-Hydroxypicolinsäure vorzuschlagen, wobei das Produkt in hoher Reinheit und mit hoher Ausbeute gebildet wird.

Die Aufgabe wurde mit einem Verfahren gemäss Patentanspruch 1 gelöst.

Erfindungsgemäß wird das Verfahren zur Herstellung von 6-Hydroxypicolinsäure also derart durchgeführt, daß man Picolinsäure und/oder deren Salze mittels Mikroorganismen der Gattung Pseudomonas, Bacillus, Alcaligenes, Aerococcus oder Rhodotorula, die mit Picolinsäure als einziger Kohlenstoff-, Stickstoff- und Energiequelle wachsen, hydroxyliert, wobei die Konzentration an Picolinsäure und/oder deren Salzen oberhalb 0,5 Gew.-% liegt, damit die 6-Hydroxypicolinsäure nicht weiter metabolisiert wird.

Als Mikroorganismen können alle der oben erwähnten Gattungen dienen, die mit Picolinsäure als einzigerKohlenstoff-, Stickstoff- und Energiequelle wachsen.

Diese Mikroorganismen können nach fachmännisch üblichen Methoden, beispielsweise aus Kläranlagen oder Erde, isoliert werden.

Zweckmässig wird zur Herstellung von 6-Hydroxypicolinsäure der Mikroorganismus Alcaligenes faecalis verwendet, der am 7.12.1990 bei der Deutschen Sammlung von Mikroorganismen und Zellkulturen GmbH, Mascheroder Weg 1b, D-3300 Braunschweig, mit der Nummer 6269 hinterlegt wurde.

Dieser Mikroorganismus war bisher noch nicht bekannt.

Eigenschaften des Stamms Alcaligenes faecalis,DSM-Nr. 6269:

| | | | |
|---|---|---|---|
| Zellform | Stäbchen | VP | − |
| Breite µm | 0,5−0,8 | | |
| Länge µm | 1,0−2,0 | ODC | − |
| Beweglichkeit | + | $NO_2$ aus $NO_3$ | − |
| Geisseln | peritrich | Denitrifikation | − |
| Gram-Reaktion | − | Phenylalanindesaminase | − |
| Lyse durch 3% KOH | + | | |
| Aminopeptidase (Cerny) | + | Levan aus Saccharose | − |
| Sporen | − | Lecithinase | − |
| Oxidase | + | Urease | − |
| | | | |
| Catalase | + | Hydrolyse von | |
| | | Stärke | − |
| Wachstum | | Gelatine | − |
| anaerob | − | Casein | − |
| 37/40°C | +/− | DNA | − |
| pH 5,6 | + | Tween 80 | − |
| Mac-Conkey-Agar | + | Aesculin | − |
| | | | |
| Pigmente | − | Tyrosin-Abbau | − |
| nicht diffundierende | − | | |
| diffundierende | − | Wuchsstoffbedarf | − |
| fluoreszierend | − | | |
| Pyocyanin | − | Substratverwertung | |
| | | Acetat | + |
| Säure aus (OF-Test) | | Adipat | − |
| Glucose aerob | − | Caprat | + |
| Glucose anaerob | − | Citrat | + |
| Xylose aerob | − | Glycolat | + |
| | | L-Lactat | + |

3

| | | | |
|---|---|---|---|
| Gas aus Glucose | – | Lävulinat | – |
| | | Malat | + |
| Säure aus (ASS) | | Malonat | + |
| Glucose | – | Phenylacetat | + |
| Fructose | – | Propionat | + |
| Xylose | – | Suberat | – |
| | | L-Arabinose | – |
| ONPG | – | Fructose | – |
| | | Glucose | – |
| ADH | – | Mannose | – |
| | | Maltose | – |
| LDC | – | Xylose | – |
| | | Ribose | – |
| VP | – | Mannit | – |
| | | Gluconat | – |
| Indol | – | 2-Ketogluconat | – |
| | | N-Acetylglucosamin | – |
| | | L-Histidin | – |
| | | L-Methionin | + |
| | | Hydroxybutyrat | + |

Für die Durchführung der Picolinsäure-Hydroxylierung ist es wünschenswert, dass die 6-Hydroxypicolinsäure nicht weiter metabolisiert wird.

Folgende Parameter sollten für die Wirtschaftlichkeit der Picolinsäure-Hydroxylierung erfüllt sein:

a) Die Zellen sollten bereits während der Wachstumsphase 6-Hydroxypicolinsäure produzieren.

b) Die Picolinsäure-Hydroxylase soll nach erfolgtem Wachstum aktiv bleiben.

c) Der Abbauweg der Picolinsäure sollte auf der Stufe der 6-Hydroxypicolinsäure gehemmt werden.

d) Das Produkt (6-Hydroxypicolinsäure) soll im Wachstumsmedium angereichert werden.

Überraschenderweise wurde nun gefunden, dass diese Parameter gleichzeitig erfüllt sind, wenn man während des Wachstums der Mikroorganismen oder auch nach der Wachstumsphase der Mikroorganismen Picolinsäure in einer solchen Konzentration zuführt, dass die 6-Hydroxypicolinsäure nicht weiter metabolisiert wird.

Wie erwähnt, wächst der Stamm mit Picolinsäure als einziger Kohlenstoff-, Stickstoff- und Energiequelle.

Die Anzucht dieses Stammes kann mit einer 0,05 bis 0,2 Gew.%igen Picolinsäure erfolgen, wobei die vorgelegte Picolinsäure vollständig metabolisiert wird.

Wird die Picolinsäure-Konzentration erhöht, wird das Zellwachstum gehemmt, und oberhalb 0,5 Gew.% Picolinsäure-Konzentration kann kein Wachstum mehr beobachtet werden.

Die Aktivität der Picolinsäure-Hydroxylase bleibt jedoch in den Zellen unverändert.

Zweckmässig wird nach der Anzucht der Mikroorganismen die Picolinsäure als 10 Gew.%ige bis gesättigte Lösung mit einer solchen Geschwindigkeit hinzugegeben, dass die Konzentration der Picolinsäure im Fermenter 10 Gew.% nicht übersteigt.

Vorzugsweise übersteigt die Konzentration der Picolinsäure im Fermenter nicht 1 Gew.%.

Zweckmässig dient die Picolinsäurelösung zusammen mit einer Alkalihydroxidlösung zur pH-Wert-Regelung des Kulturmediums.

Als Alkalihydroxid kann beispielsweise Natriumhydroxid oder Kaliumhydroxid verwendet werden.

Als Kulturmedium können die in der Fachwelt üblichen verwendet werden, vorzugsweise wird ein Mineralsalzmedium verwendet, dessen Zusammensetzung in Tabelle 2 angegeben ist.

4

Zweckmässig beträgt der Sauerstoffgehalt im Kulturmedium während der Hydroxylierung bis zu 90% der maximalen Sättigung, vorzugsweise liegt der Sauerstoffgehalt in einem Bereich von 0,1 bis 50% der maximalen Sättigung.

Die Hydroxylierung der Picolinsäure kann während oder nach der Wachstumsphase erfolgen.

Der pH-Wert liegt zweckmässig während oder nach der Wachstumsphase zwischen pH 4 und 10, vorzugsweise zwischen 5 und 9.

Die Hydroxylierung erfolgt zweckmässig bei einer Temperatur von 10 bis 60°C, vorzugsweise von 15 bis 40°C.

Um die Produktkonzentration zusätzlich zu steigern, wird in einer vorzugsweisen Ausführungsform der Erfindung nach der Wachstumsphase der Mikroorganismen ein Alkalisalz der Picolinsäure zugeführt, wobei die Zufuhr des Picolinsäurealkalisalzes von der Sauerstoffpartialdruck-Regelung im Fermenter gesteuert wird.

Das Alkalisalz der Picolinsäure wird zweckmässig in einer solchen Geschwindigkeit hinzugegeben, dass die Konzentration des Picolinsäuresalzes im Fermenter 10 Gew.% nicht übersteigt, vorzugsweise, dass sie 1 Gew.% nicht übersteigt.

Beispiel 1

Isolation von Alcaligenes faecalis (DSM 6269)

Aerobe Picolinsäure-metabolisierende Mikroorganismen wurden in A + N-Medium (Tabelle 1) mit Zusatz von 0,1% (w/v) Picolinsäure als einzigerKohlenstoff- und Energiequelle angereichert. Die allgemeinen Techniken zur Isolation von Mikroorganismen sind beispielsweise in "G.Drews, Mikrobiologisches Praktikum, 4.Aufl., Springer Verlag, 1983, S. 1 - 84" beschrieben. Als Inokulum wurden Proben aus dem Erdreich oder Kläranlagen verwendet. Die Anreicherungen wurden in Schüttelkolben bei 30°C angezogen. Nach dreimaligem Ueberimpfen in frisches Medium wurden die Anreicherungen auf dem gleichen Medium mit Zusatz von 16 g Agar pro Liter ausgestrichen und bei 30°C inkubiert. Nach mehrmaligem Ausstreichen auf Agarmedium konnten Reinkulturen isoliert werden.

Beispiel 2

Hydroxylierung in der Wachstumsphase

Alcaligenes faecalis (DSM 6269) wurde aerob in einem Mineralsalzmedium (Tabelle 2) mit Picolinsäure als einziger Kohlenstoff-, Stickstoff- und Energiequelle bei pH 7 und bei einer Temperatur von 30°C angezogen.

Zur Anzucht diente ein 20-Liter-Fermenter mit einem Arbeitsvolumen von ca. 15 l.

Zur pH-Regelung wurde eine 4,06 mol/l (50% w/v, ca. 2 l) Picolinsäurelösung und 3 mol/l (12% w/v, < 20 ml) Natriumhydroxidlösung innerhalb 27 h zudosiert.

Danach wurden in der Fermentationslösung 0,14 mol/l (2% w/v) 6-Hydroxy-picolinsäure und 16 mmol/l (0,18% w/v) Picolinsäure nachgewiesen. Zu diesem Zeitpunkt betrug der Sauerstoffgehalt bei einem Luftdurchsatz von 30 l/min und bei einer Rünhrgeschwindigkeit von 750 Umdrehungen/min 1% Sättigung.

Hydroxylierung nach der Wachstumsphase

Nach der Wachstumsphase wurde eine 2,7 mol/l (47% w/v, ca. 2,5 l) Natriumpicolinatlösung (pH 7) innerhalb 15 h in den Fermenter zudosiert.

Die Dosiergeschwindigkeit der Natriumpicolinatlösung wurde von der Sauerstoffpartialdruckregelung des Fermenters so gesteuert, dass der Sauerstoffgehalt eine Sättigung von 20% nicht überschritt, wobei die Konzentration an Picolinat ca. 20 mmol/l (0,22% w/v) betrug. Bei einer Konzentration von 0,7 mol/l (9,8% w/v) 6-Hydroxypicolinsäure und einer Fermentationsdauer von insgesamt 42 h wurde der Ansatz abgebrochen. Für den Ansatz wurden insgesamt 17,8 mol (2190 g) Picolinsäure in Form von freier Säure und als Natriumsalz eingesetzt. Aus Picolinsäure bzw. deren Natriumsalz konnten 13,3 mol (1850 g) 6-Hydroxypicolinsäure in kristalliner Form nach dem Ansäuern der zellfreien Lösung isoliert werden, was einer Ausbeute von 74%, bezogen auf eingesetzte Picolinsäure,entsprach. Gemäss HPLC-Analyse betrug die Reinheit der 6-Hydroxypicolinsäure mehr als 95%.

Im klaren Filtrat waren 0,13 mol (18,5 g) 6-Hydroxypicolinsäure, entsprechend 0,75% der eingesetzten Picolinsäure,und 0,3 mol (37 g) Picolinsäure, entsprechend 1,7% der eingesetzten Picolinsäure, nachweisbar.

Tabelle 1

| A + N-Medium | |
|---|---|
| Zusammensetzung: | Konzentration (mg/l) |
| $(NH_4)_2SO_4$ | 2000 |
| $Na_2HPO_4$ | 2000 |
| $KH_2PO_3$ | 1000 |
| NaCl | 3000 |
| $MgCl_2 \cdot 6H_2O$ | 400 |
| $CaCl_2 \cdot 2H_2O$ | $14 \cdot 5$ |
| $FeCl_3 \cdot 6H_2O$ | $0 \cdot 8$ |
| Pyridoxal-Hydrochlorid | $10 \cdot 10^{-3}$ |
| Riboflavin | $5 \cdot 10^{-3}$ |
| Nicotinsäureamid | $5 \cdot 10^{-3}$ |
| Thiamin-Hydrochlorid | $2 \cdot 10^{-3}$ |
| Biotin | $2 \cdot 10^{-3}$ |
| Panthotensäure | $5 \cdot 10^{-3}$ |
| p-Aminobenzoat | $5 \cdot 10^{-3}$ |
| Folsäure | $2 \cdot 10^{-3}$ |
| Vitamin B12 | $5 \cdot 10^{-3}$ |
| $ZnSO_4 \cdot 7H_2O$ | $100 \cdot 10^{-3}$ |
| $MnCl_2 \cdot 4H_2O$ | $90 \cdot 10^{-3}$ |
| $H_3BO_3$ | $300 \cdot 10^{-3}$ |
| $CoCl_2 \cdot 6H_2O$ | $200 \cdot 10^{-3}$ |
| $CuCl_2 \cdot 2H_2O$ | $10 \cdot 10^{-3}$ |
| $NiCl_2 \cdot 6H_2O$ | $20 \cdot 10^{-3}$ |
| $Na_2MoO_4 \cdot 2H_2O$ | $30 \cdot 10^{-3}$ |
| $EDTANa_2 \cdot 2H_2O$ | $5 \cdot 10^{-3}$ |
| $FeSO_4 \cdot 7H_2O$ | $2 \cdot 10^{-3}$ |
| (pH der Lösung wurde nach der Zugabe von Picolinsäure auf 7,0 eingestellt) | |

Tabelle 2

| Mineralsalzmediumzusammensetzung: | |
|---|---|
| - Picolinsäure | 2 g/l |
| - $MgCl_2 \cdot 6\,H_2O$ | 0,8 g/l |
| - $CaCl_2$ | 0,16 g/l |
| - $Na_2SO_4$ | 0,25 g/l |
| - $KH_2SO_4$ | 0,4 g/l |
| - $Na_2HPO_4$ | 0,9 g/l |
| - Spurenelemente | 1 ml/l |
| Zusammensetzung der Spurenelementlösung: | |
| - Picolinsäure | 200 g/l |
| - NaOH | 65 g/l |
| - $ZnSO_4 \cdot 7H_2O$ | 9 g/l |
| - $MnCl_2 \cdot 4H_2O$ | 4 g/l |
| - $H_3BO_3$ | 2,7 g/l |
| - $CoCl_2 \cdot 6H_2O$ | 1,8 g/l |
| - $CuCl_2 \cdot 2H_2O$ | 1,5 g/l |
| - $NiCl_2 \cdot 6H_2O$ | 0,18 g/l |
| - $Na_2MoO_4 \cdot 2H_2O$ | 0,2 g/l |
| - $FeSO_4 \cdot 7H_2O$ | 30 g/l |
| (Der pH-Wert der Lösung wurde auf 7,0 eingestellt) | |

**Patentansprüche**

1. Mikrobiologisches Verfahren zur Herstellung von 6-Hydroxypicolinsäure, dadurch gekennzeichnet, daß man Picolinsäure und/oder deren Salze mittels Mikroorganismen der Gattung Pseudomonas, Bacillus, Alcaligenes, Aerococcus oder Rhodotorula, die mit Picolinsäure als einziger Kohlenstoff-, Stickstoff- und Energiequelle wachsen, hydroxyliert, wobei die Konzentration an Picolinsäure und/oder deren Salzen oberhalb 0,5 Gew.-% liegt, um den Abbau der Picolinsäure auf der Stufe der 6-Hydroxypicolinsäure zu hemmen.

2. Verfahren nach Patentanspruch 1, dadurch gekennzeichnet, daß man die Hydroxylierung mit Alcaligenes faecalis, hinterlegt bei der DSM unter der Nummer 6269, oder dessen Mutanten, die mit Picolinsäure als einziger Kohlenstoff-, Stickstoff- und Energiequelle wachsen, durchführt.

3. Verfahren nach mindestens einem der Patentansprüche 1 - 2, dadurch gekennzeichnet, daß die Konzentration an Picolinsäure und/oder deren Salzen so gewählt wird, daß sie 10 Gew.-% nicht übersteigt.

4. Verfahren nach mindestens einem der Patentansprüche 1 - 3, dadurch gekennzeichnet, daß man die Hydroxylierung bei einer Temperatur von 10 bis 60°C und bei einem pH von 4 bis 10 durchführt.

5. Mikroorganismus Alcaligenes faecalis, hinterlegt bei der DSM mit der Nummer 6269, und dessen Mutanten, die mit Picolinsäure als einziger Kohlenstoff-, Stickstoff- und Energiequelle wachsen.

**Claims**

1. A microbiological process for the production of 6-hydroxypicolinic acid, characterized in that picolinic acid and/or the salts thereof are hydroxylated by means of microorganisms of the genus Pseudomonas, Bacillus, Alcaligenes, Aerococcus or Rhodotorula growing with picolinic acid as the sole source of carbon, nitrogen and energy, the concentration of picolinic acid and/or of the salts thereof being above 0.5 % by weight in order to inhibit degradation of the picolinic acid at the stage of 6-hydroxypicolinic acid.

7

2. Process according to claim 1, characterized in that the hydroxylation is carried out with <u>Alcaligenes faecalis</u>, deposited at the DSM under the number 6269, or with mutants thereof growing with picolinic acid as the sole source of carbon, nitrogen and energy.

3. Process according to at least one of claims 1 - 2, characterized in that the concentration of picolinic acid and/or of the salts thereof is selected so as not to exceed 10 % by weight.

4. Process according to at least one of claims 1 - 3, characterized in that the hydroxylation is carried out at a temperature of from 10 to 60 °C and at a pH of from 4 to 10.

5. Microorganism <u>Alcaligenes faecalis</u>, deposited at the DSM under the number 6269, and mutants thereof growing with picolinic acid as the sole source of carbon, nitrogen and energy.

**Revendications**

1. Procédé microbiologique de préparation d'acide 6-hydroxypicolinique, caractérisé en ce que l'on hydroxyle l'acide picolinique et/ou ses sels à l'aide de microorganismes du genre *Pseudomonas, Bacillus, Alcaligenes, Aerococcus* ou *Rhodotorula* qui se développent avec de l'acide picolinique comme unique source de carbone, d'azote et d'énergie, la concentration en acide picolinique et/ou en ses sels étant supérieure à 0,5 % en masse pour empêcher la dégradation de l'acide picolinique au-delà du stade de l'acide 6-hydroxypicolinique.

2. Procédé selon la revendication 1, caractérisé en ce que l'on effectue l'hydroxylation avec *Alcaligenes faecalis*, déposée auprès du DSM sous le numéro 6269, ou avec ses mutants qui se développent avec de l'acide picolinique comme unique source de carbone, d'azote et d'énergie.

3. Procédé selon l'une au moins des revendications 1 - 2, caractérisé en ce que l'on choisit la concentration d'acide picolinique et/ou de ses sels de façon qu'elle ne dépasse pas 10 % en masse.

4. Procédé selon l'une au moins des revendications 1 - 3, caractérisé en ce que l'on effectue l'hydroxylation à une température de 10 à 60 °C et à un pH de 4 à 10.

5. Microorganisme *Alcaligenes faecalis*, déposé auprès du DSM sous le numéro 6269, et ses mutants qui se développent avec de l'acide picolinique comme unique source de carbone, d'azote et d'énergie.